# EUROPÄISCHE PATENTSCHRIFT

(11) **EP 4 007 912 B1**
(45) Veröffentlichungstag und Bekanntmachung des Hinweises auf die Patenterteilung: **30.08.2023**
(21) Anmeldenummer: 20754179.8
(22) Anmeldetag: 27.07.2020
(51) Int. Cl.: G01N 27/416, G01N 1/38

(54) **MOBILE ANLAGE FÜR DAS KALIBRIEREN, VERIFIZIEREN UND/ODER JUSTIEREN EINES SENSORS UND VERFAHREN ZUM KALIBRIEREN, VERIFIZIEREN UND/ODER JUSTIEREN EINES SENSORS**
MOBILE SYSTEM FOR CALIBRATING, VERIFYING AND/OR ADJUSTING A SENSOR AND METHOD FOR CALIBRATING, VERIFYING AND/OR ADJUSTING A SENSOR
SYSTÈME MOBILE ET PROCÉDÉ D'ÉTALONNAGE, DE VÉRIFICATION ET/OU DE RÉGLAGE D'UN CAPTEUR

(30) Priorität: 02.08.2019 DE 102019120897
(43) Veröffentlichungstag der Anmeldung: 08.06.2022
(73) Patentinhaber: Endress+Hauser Group Services AG, 4153 Reinach (CH)
(72) Erfinder: HELLEIN, Bernhard, 3441 Pixendorf (AT)
(74) Vertreter: Hahn, Christian
(86) Internationale Anmeldenummer: PCT/EP2020/071073
(87) Internationale Veröffentlichungsnummer: WO 2021/023537

(56) Entgegenhaltungen:
- DE-A1-102016 121 439
- JP-A- H09 288 054
- US-A- 5 403 497

## Beschreibung

Die Erfindung betrifft eine mobile Anlage für das Kalibrieren, Verifizieren und/oder Justieren eines Sensors zur Bestimmung der Prozessgröße eines Prozessmediums. Ferner betrifft die Erfindung ein Verfahren zum Kalibrieren, Verifizieren und/oder Justieren eines Sensors.

Bei der mit dem Sensor bestimmbaren Prozessgröße handelt es sich insbesondere um eine Analysemessgröße, welche von der Konzentration einer oder mehrerer Substanzen in dem Messmedium (sog. Analyt bzw. Analyte) abhängig ist, bspw. eine Konzentration oder eine Aktivität oder eine Summe von Konzentrationen mehrerer Analyten. Derartige Sensoren werden in der Labor- und Prozessmesstechnik in vielen Bereichen der Chemie, Biochemie, Pharmazie, Biotechnologie, Lebensmitteltechnologie, Wasserwirtschaft und Umweltmesstechnik bei der Analyse von Messmedien eingesetzt. Bei dem Sensor zur Bestimmung der Prozessgröße bzw. Analysemessgröße handelt es sich beispielsweise um einen potentiometrischen (bspw. ionenselektive Elektrode (ISE), etwa die bekannte pH-Glaselektrode) oder amperometrischen Sensor (bspw. amperometrische Desinfektionssensor). Weitere Beispiele Sensoren sind solche auf der Basis von Elektrolyt-Isolator-Halbleiter-Schichtstapeln (englisch: electrolyte-insulator-semiconductor, kurz: EIS), z.B. ISFET-Sensoren, induktiv oder kapazitiv arbeitende Leitfähigkeitssensoren oder (spektro)-photometrisch arbeitende Sensoren, wie etwa Trübungssensoren. Die Anmelderin vertreibt eine Vielzahl derartiger Sensoren zur Bestimmung einer Analysemessgröße in den unterschiedlichsten Ausgestaltungen.

Derartige Sensoren müssen üblicherweise kalibriert, verifiziert und/oder justiert werden. Hierbei wird bspw. ein als Referenz dienender Referenzsensor bzw. ein damit ermittelter, als korrekt angenommener Messwert als Referenzwert verwendet. Unter dem Kalibrieren versteht man dabei üblicherweise das Feststellen einer Abweichung des mit dem Sensors gemessenen Messwerts von dem als korrekt angenommenen Referenzwert. Das Verifizieren umfasst zusätzlich das Ermitteln der Abweichung und deren Bewertung. Unter dem Justieren versteht man das Anpassen des Sensors in der Weise, dass dessen Messwert mit dem Referenzwert übereinstimmt. Das Kalibrieren, Verifizieren und/oder Justieren geschieht in der Regel zumindest bei der Inbetriebnahme des Sensor oder gegebenenfalls auch wiederholt - etwa in regelmäßigen Kalibrierintervallen -, wenn bspw. eine alterungsbedingte Drift des Sensors anzunehmen ist.

In der Praxis werden bei dem Kalibrieren, Verifizieren und/oder Justieren eines Sensors oftmals mehrere, voneinander verschiedene Referenzwerte verwendet (auch: Mehrpunkt-Kalibrierung). Hierzu werden mehrere Kalibrierstandards verwendet, welche voneinander verschiedene, insb. vorgegebene, Werte für die mit dem Sensor (bzw. dem Referenzsensor) bestimmbare Prozessgröße, insb. Analysemessgröße aufweisen. Der zu kalibrierende, zu verifizierende und/oder zu justierende Sensor und der Referenzsensor werden dann bspw. aufeinanderfolgend mit den Kalibrierstandards in Kontakt gebracht. Beispielsweise liegen die Kalibrierstandards als eine Reihe von Kalibrierflüssigkeiten (bspw. Kalibrierlösungen, etwas Pufferlösungen) vor, in welche der zu kalibrierende, zu verifizierende und/oder zu justierende Sensor sowie ggf. auch der Referenzsensor nacheinander eingebracht werden.

Eine Anlage zum Kalibrieren eines photometrischen Sensors mit einer Pumpe und einer Förderleitung zum Fördern einer Kalibrierflüssigkeit sowie mit Filtern zum Reinigen der Kalibrierflüssigkeit für eine Nullpunktmessung ist in der Offenlegungsschrift JP H09 288054 A offenbart.

Gegebenenfalls wird in der Praxis der Referenzsensor nur bei der Herstellung der Kalibrierflüssigkeit und nicht beim Kalibrieren, Verifizieren und/oder Justieren des Sensors eingesetzt. Der mit dem Referenzsensor bei der Herstellung der Kalibrierflüssigkeit erfasste Messwert für die Prozessgröße wird der Kalibrierflüssigkeit zugeordnet. Aufgrund einer oftmals vorliegenden Temperaturabhängigkeit wird in diesem Fall bei einem späteren Kalibrieren, Verifizieren und/oder Justieren zusätzlich die Temperatur gemessen. Für den Fall, dass sich die Temperatur bei der Herstellung der Kalibrierflüssigkeit von der Temperatur beim Kalibrieren, Verifizieren und/oder Justieren des Sensors unterscheiden, wird diese Temperaturabhängigkeit rechnerisch korrigiert.

Dies gestaltet sich als relativ aufwändig, nicht zuletzt auch, da der oder die Sensoren hierbei jeweils einer gründlichen Reinigung unterzogen werden müssen, um eine gegenseitige Kontaminierung der Kalibrierflüssigkeiten untereinander durch etwaige Rückstände an dem/den Sensor/en wirksam zu verhindern.

Der Erfindung liegt daher die Aufgabe zugrunde, das Kalibrieren, Verifizieren und/oder Justieren eines Sensors zu vereinfachen.

Die Aufgabe wird gelöst durch eine mobile Anlage für das Kalibrieren, Verifizieren und/oder Justieren eines Sensors sowie durch ein Verfahren zum Kalibrieren, Verifizieren und/oder Justieren eines Sensors zur Bestimmung einer von einer Salzkonzentration abhängigen Prozessgröße eines Prozessmediums, bei dem eine mobile Anlage verwendet wird.

Bezüglich der mobilen Anlage wird die Aufgabe gelöst durch eine mobile Anlage für das Kalibrieren, Verifizieren und/oder Justieren eines Sensors zur Bestimmung einer von einer Salzkonzentration abhängigen Prozessgröße eines Prozessmediums, umfassend einen Förderkreislauf, der zum Fördern einer darin eingebrachten Kalibrierflüssigkeit ausgestaltet ist, aufweisend:
- eine Fluidleitung;
- eine Bypass-Fluidleitung, die über eine erste Verbindungsstelle und eine zweite Verbindungsstelle in einem Bypass zu einem ersten Abschnitt der Fluidleitung mit der Fluidleitung verbunden ist,
   mit einer Filtereinheit, die zum fortlaufenden Entsalzen eines durch die Bypass-Fluidleitung geförderten Anteils der Kalibrierflüssigkeit ausgestaltet ist und;
- eine in der Fluidleitung angeordnete Pumpe, die dazu ausgestaltet ist, die Kalibrierflüssigkeit in einer Förderrichtung durch den Förderkreislauf zu fördern;
- eine in einem vom ersten Abschnitt verschiedenen zweiten Abschnitt der Fluidleitung angeordnete Kalibrierstelle, an der der zu kalibrierende, zu verifizierende und/oder zu justierende Sensor einbringbar ist sowie ein zur Messung der Prozessgröße dienender Referenzsensor in den Förderkreislauf eingebracht ist und/oder in einem vom ersten Abschnitt verschiedenen zweiten Abschnitt der Fluidleitung angeordnete Anschlüsse, mittels derer zumindest eine als Kalibrierstelle dienende Wechselarmatur in den Förderkreislauf integriert ist, wobei mit der zumindest einen Wechselarmatur der zu kalibrierende, zu verifizierende und/oder zu justierende Sensor einbringbar ist sowie ein zur Messung der Prozessgröße dienender Referenzsensor in den Förderkreislauf eingebracht ist, und
- eine einstellbare Ventileinheit, mittels derer ein Verhältnis des durch die Bypass-Fluidleitung geförderten Anteils der Kalibrierflüssigkeit zu einem durch den ersten Abschnitt der Fluidleitung geförderten Anteil der Kalibrierflüssigkeit einstellbar ist,
wobei mittels einer Einstellung, insb. Regelung, der Ventileinheit die an der Kalibrierstelle vorliegende Prozessgröße auf zumindest einen vorgebbaren Referenzwert für die Prozessgröße einstellbar, insb. regelbar, ist.

Die Prozessgröße der Kalibrierflüssigkeit ist gemäß Erfindung mittels der Einstellung der Ventileinheit auf den zumindest einen vorgebbaren Referenzwert einstellbar. Die Einstellung der Ventileinheit bestimmt den die Bypass-Leitung sowie den den ersten Abschnitt der Fluidleitung durchfließenden Anteil der Kalibrierflüssigkeit. Auf diese Weise wird der in der Filtereinheit der Bypass-Leitung fortlaufend entsalzte Anteil und damit der letztendlich an der Kalibrierstelle vorliegende Salzgehalt der Kalibrierflüssigkeit eingestellt. Da die Prozessgröße von einer Salzkonzentration der Kalibrierflüssigkeit abhängt, ist hierüber auch der Referenzwert einstellbar.

Bei der Filtereinheit handelt es sich insbesondere um eine Entsalzungsanlage, bspw. einen sogenannten Mischbettfilter. Der Bypass des Förderkreislaufs ist hier dadurch gebildet, dass sich an der ersten Verbindungsstelle der zweite Abschnitt der Fluidleitung in den ersten Abschnitt und die Bypass-Fluidleitung verzweigt. Diese Bypass-Fluidleitung und der erste Abschnitt münden an der zweiten Verbindungsstelle wieder in den zweiten Abschnitt, wodurch der Förderkreislauf geschlossen wird.

Die Vorteile der Erfindung sind die folgenden:
- Die mobile Anlage ermöglicht die nachträgliche Einstellung der Prozessgröße auf den zumindest vorgebbaren Referenzwert. "Nachträglich" bedeutet, dass die Kalibrierflüssigkeit im Förderkreislauf verbleibt. Auch der Sensor und der Referenzsensor verbleiben direkt in Kontakt mit der Kalibrierflüssigkeit bei der Einstellung der Prozessgröße auf den zumindest einen Referenzwert. Dadurch ist keine vorherige Reinigung der Sensoren mehr vonnöten. Vorteilhaft können auch nacheinander mehrere verschiedene Referenzwerte eingestellt werden.
- Durch die erfindungsgemäße Lösung sind insbesondere auch Referenzwerte der Kalibrierflüssigkeit einstellbar, die sehr empfindlich auf den Salzgehalt der Kalibrierflüssigkeit an der Kalibrierstelle sind. Dagegen ist ein Kalibrieren, Verifizieren und/oder Justieren bspw. eines Leitfähigkeitssensors mit einem Messbereich unterhalb von 50 µS/cm mit den aus dem Stand der Technik bekannten Kalibrierlösungen schwierig bis nicht praktikabel, aufgrund deren hoher Empfindlichkeit gegenüber Verschmutzungen.
- Mit der mobilen Anlage lässt sich eine höhere Genauigkeit erreichen, im Vergleich zu einem Kalibrieren, Verifizieren und/oder Justieren, bei dem der Referenzsensor nur bei der Herstellung von Kalibrierflüssigkeiten eingesetzt wird und eine Temperaturabhängigkeit der Prozessgröße mittels einer Temperaturmessung rechnerisch noch korrigiert werden muss. In letzten Fall liegt nämlich eine sich fortpflanzende Ungenauigkeit der Temperaturmessung vor, wohingegen bei der erfindungsgemäßen mobilen Anlage nur die Ungenauigkeit der Messung mit dem Referenzsensor vorliegt.
- Es handelt sich um eine mobile Anlage. Insbesondere ist bevorzugt die Anlage kompakt als eine tragbare mobile Anlage, bspw. mit einem Gesamtgewicht unterhalb 10kg, ausgebildet. Dadurch wird vorteilhaft eine Vor-Ort-Kalibrierung eines Sensors an seinem bestimmungsgemäßen Einsatzort ermöglicht.

In einer Ausgestaltung der mobilen Anlage umfasst diese ein in dem zweiten Abschnitt der Fluidleitung angeordnetes Behältnis, das mit der Fluidleitung verbunden ist, mittels eines Einlasses zum Einströmen der Flüssigkeit aus dem zweiten Abschnitt der Fluidleitung in das Behältnis und mittels eines Auslasses zum Ausströmen der Flüssigkeit aus dem Behältnis in den zweiten Abschnitt der Fluidleitung.

Durch das Behältnis ist die Kalibrierflüssigkeit in die mobile Anlage einfüllbar und ein für das beim Kalibrieren, Verifizieren und/oder Justieren des Sensors benötigtes Volumen an Kalibrierflüssigkeit möglichst einfach bereitstellbar. Das Behältnis umfasst hierzu typischerweise ein Volumen kleiner als 10l, bspw. 0,5 bis 5l.

In einer Ausgestaltung der mobilen Anlage bildet das Behältnis die Kalibrierstelle, so dass beim Kalibrieren, Verifizieren und/oder Justieren des Sensors der zu kalibrierende, zu verifizierende und/oder zu justierende Sensor sowie der Referenzsensor in die in dem Behältnis enthaltene Kalibrierflüssigkeit eintauchen.

In einer weiteren Ausgestaltung der mobilen Anlage handelt es sich um ein offenes Behältnis, wobei ein Gas, das in der das offene Behältnis umgebenden Umgebungsluft enthalten ist, mittels einer chemischen Reaktion zwischen dem Gas und der in der im offenen Behältnis enthaltenen Kalibrierflüssigkeit Salze in der Kalibrierflüssigkeit bildet. Dadurch wird also ausgehend von einer Ausgangs-Salzkonzentration die Salzkonzentration der Kalibrierflüssigkeit an der Kalibrierstelle erhöht. In Kombination mit der erfindungsgemäßen fortlaufenden Entsalzung durch die Filtereinheit in der Bypass-Leitung wird hierdurch sowohl eine Erhöhung, als auch oder eine Erniedrigung der Salzkonzentration an der Kalibrierstelle ermöglicht.

Selbstverständlich ist im Rahmen der Erfindung auch ein geschlossenes Behältnis oder ein Förderkreislauf ohne Behältnis möglich, wobei in diesem Fall nur die Erniedrigung der Salzkonzentration an der Kalibrierstelle möglich ist.

In einer besonders bevorzugten Weiterbildung der mobilen Anlage steuert ein einstellbarer Regler der Ventileinheit die Ventileinheit dazu, dass die an der Kalibrierstelle vorliegende Prozessgröße aufeinanderfolgend unterschiedliche vorgebbare Referenzwerte für die Prozessgröße, insb. zumindest zwei unterschiedliche vorgebbare Referenzwerte für die Prozessgröße, aufweist, während die Kalibrierflüssigkeit, der zu kalibrierende, zu verifizierende und/oder zu justierende Sensor und der als Referenz dienende Referenzsensor in dem Förderkreislauf verbleiben.

Mit der mobilen Anlage ist also insb. das aufeinanderfolgende Ansteuern der verschiedenen Referenzwerte möglich, ohne dass wie beim Hantieren mit den aus dem Stand der Technik bekannten Kalibrierlösungen zwischendurch eine aufwändige Reinigung des Sensors und ggf. des Referenzsensors vonnöten ist. Sensor und Referenzsensor verbleiben erfindungsgemäß ständig in Kontakt mit der Kalibrierflüssigkeit. Diese weist nur durch das Verstellen der Ventileinheit aufeinanderfolgend die unterschiedlichen Referenzwerte auf. Eine Mehrpunkt-Kalibrierung gestaltet sich hierdurch als besonders einfach.

In einer besonders bevorzugten Ausgestaltung der mobilen Anlage handelt es sich bei der mobilen Anlage um eine mobile Anlage für das Kalibrieren, Verifizieren und/oder Justieren eines Leitfähigkeitssensors, der insbesondere einen Messbereich aufweist, der durch einen maximalen Leitwert kleiner als 50 µS/cm, vorzugsweise kleiner als 10 µS/cm, begrenzt ist. Wie vorstehend erwähnt ist bei einem derartigen Messbereich ein Kalibrieren, Verifizieren und/oder Justieren eines Leitfähigkeitssensors mit Kalibrierlösungen sehr anspruchsvoll. Dies gilt insb. im Falle einer Vor-Ort-Kalibrierung bei der in der Regel keine Laborbedingungen erreicht werden können. Beispielsweise ist das Einstellen eines Referenzwerts kleiner als 1 µS/cm mit Kalibrierlösungen in diesem Fall quasi ausgeschlossen.

In einer Ausgestaltung der mobilen Anlage ist die Pumpe in dem zweiten Abschnitt der Fluidleitung angeordnet. Dadurch bewirkt die Pumpe eine zuverlässige Förderung durch den gesamten Förderkreislauf, d.h. den zweiten Abschnitt der Fluidleitung, sowie die zwischen der ersten und der zweiten Verbindungstelle im Bypass geschaltete Bypass-Leitung und den ersten Abschnitt der Fluidleitung.

In einer Ausgestaltung der mobilen Anlage ist die Pumpe angrenzend zu der ersten Verbindungstelle angeordnet, in Bezug auf die Förderrichtung der Pumpe im Wesentlichen stromaufwärts der ersten Verbindungsstelle.

In einer weiteren Ausgestaltung der mobilen Anlage ist die Ventileinheit an der zweiten Verbindungsstelle angeordnet.

Bei der Ventileinheit handelt es sich bspw. um ein einstellbares 2/3-Wege Ventil oder eine Einheit von mehreren miteinander zusammenwirkenden Ventilen. Bevorzugt ist die Ventileinheit nach der Erfindung an der zweiten Verbindungsstelle angeordnet. Dadurch liegt in der Bypass-Leitung und dem ersten Abschnitt der Fluidleitung derselbe Druck vor. Selbstverständlich ist es im Rahmen der Erfindung aber auch möglich, die Ventileinheit an der ersten Verbindungsstelle anzuordnen und/oder an dieser eine weitere Ventileinheit vorzusehen.

In einer weiteren Ausgestaltung der mobilen Anlage ist als Kalibrierflüssigkeit zumindest teilentsalztes, insb. vollentsalztes Wasser, in den Förderkreislauf eingefüllt.

In einer weiteren Ausgestaltung der mobilen Anlage ist der Regler der Ventileinheit und/oder die Ventileinheit selbst manuell betätigbar. Das Kalibrieren, Verifizieren und/oder Justieren des Sensors erfolgt also z.B. dadurch, dass ein Anwendungstechniker den Regler bzw. die Ventieleinheit manuell einstellt bzw. regelt, bis eine mit dem Referenzsensor ermittelte Prozessgröße mit dem bei dem Kalibrieren, Verifizieren und/oder Justieren verwendeten vorgebbaren Referenzwert überstimmt. Dies erfolgt im Falle einer Mehrpunkt-Kalibrierung in aufeinanderfolgenden Schritten ggf. wiederholt.

In einer weiteren Ausgestaltung der mobilen Anlage umfasst diese eine Regel-/Auswerteeinheit, die den Regler umfasst, wobei die Regel-/Auswerteeinheit mit der Ventileinheit verbunden und mit dem Referenzsensor verbindbar ist. Die Regel-/Auswerteeinheit ermöglicht eine (teil-)automatische Regelung der Regel-/Auswerteeinheit, da sie zur Regelung Ventileinheit ausgestaltet ist. Dabei kann die mobile Anlage selbstverständlich sowohl zur manuellen, als auch zur (teil-)automatische Regelung ausgestaltet sein.

In einer bevorzugten Ausgestaltung der mobilen Anlage sind deren Komponenten, insb. die Fluidleitung, die Bypass-Fluidleitung, die Filtereinheit, das Behältnis, die Pumpe und die Ventileinheit, als ineinander zusammensteckbare Komponenten ausgestaltet. Durch die zusammensteckbare mobile Anlage wird ein besonders einfacher, im wesentlichen Werkzeug-freier (d.h. ohne das Verwenden zusätzlicher Werkzeuge) Aufbau der mobilen Anlage und/oder Austausch deren Komponenten ermöglicht. Bspw. ist ein Austausch der sich im Betrieb allmählich zusetzenden Filtereinheit sehr einfach möglich.

In einer weiteren Ausgestaltung der mobilen Anlage weisen die Fluidleitung und/oder die Bypass-Leitung einen thermoplastischen Werkstoff, insb. Polytetrafluorethylen (PTFE), auf. Sie können bspw. aus diesem Werkstoff bestehen, oder mind. teilweise damit ausgekleidet sein.

Bezüglich des Verfahrens wird die Aufgabe gelöst durch ein Verfahren zum Kalibrieren, Verifizieren und/oder Justieren eines Sensors zur Bestimmung einer von einer Salzkonzentration abhängigen Prozessgröße eines Prozessmediums,
wobei eine mobile Anlage verwendet wird, umfassend einen Förderkreislauf, der zum Fördern einer darin eingebrachten Kalibrierflüssigkeit ausgestaltet ist, aufweisend:
   - eine Fluidleitung;
   - eine Bypass-Fluidleitung, die über eine erste Verbindungsstelle und eine zweite Verbindungsstelle in einem Bypass zu einem ersten Abschnitt der Fluidleitung mit der Fluidleitung verbunden ist,
mit einer Filtereinheit, die zum fortlaufenden Entsalzen eines durch die Bypass-Fluidleitung geförderten Anteils der Kalibrierflüssigkeit ausgestaltet ist und;
   - eine in der Fluidleitung angeordnete Pumpe, die dazu ausgestaltet ist, die Kalibrierflüssigkeit in einer Förderrichtung durch den Förderkreislauf zu fördern;
   - eine einstellbare Ventileinheit, mittels derer ein Verhältnis des durch die Bypass-Fluidleitung geförderten Anteils der Kalibrierflüssigkeit zu einem durch den ersten Abschnitt der Fluidleitung geförderten Anteils der Kalibrierflüssigkeit einstellbar ist,
wobei das Verfahren die Schritte umfasst:
   - Einbringen des zu kalibrierenden, zu verifizierenden und/oder zu justierenden Sensor sowie eines zur Messung der Prozessgröße dienenden Referenzsensors in den Förderkreislauf an einer im zweiten Abschnitt der Fluidleitung angeordneten Kalibrierstelle und/oder in zumindest eine, als Kalibrierstelle dienende Wechselarmatur, die mittels im zweiten Abschnitt der Fluidleitung angeordneter Anschlüsse in den Förderkreislauf der mobilen Anlage integriert ist;
   - Einstellen, insb. Regeln, der Ventileinheit derart, dass die an der Kalibrierstelle vorliegende Prozessgröße einen vorgebbaren Referenzwert für die Prozessgröße aufweist.

In einer Ausgestaltung des Verfahrens erfolgt das Einstellen, insb. Regeln, der Ventileinheit mit dem Ziel, dass ein mit dem Referenzsensor ermittelter Messwert der Prozessgröße mit dem vorgebbaren Referenzwert übereinstimmt.

In einer Weiterbildung des Verfahrens umfasst dieses:
- Wiederkehrendes Einstellen, insb. Regeln, der Ventileinheit derart, dass die an der Kalibrierstelle vorliegenden Prozessgröße zu aufeinanderfolgenden Zeitpunkten unterschiedliche vorgebbare Referenzwerte für die Prozessgröße, insb. zumindest zwei unterschiedliche vorgebbare Referenzwerte für die Prozessgröße aufweist, wobei die Kalibrierflüssigkeit, der zu kalibrierende, zu verifizierende und/oder zu justierende Sensor und der als Referenz dienende Referenzsensor zu den aufeinanderfolgenden Zeitpunkten während dem wiederkehrenden Einstellen, insb. Regeln, der Ventileinheit in dem Förderkreislauf verbleiben.

Die Erfindung wird anhand der nachfolgenden, nicht maßstabsgetreuen Figuren näher erläutert, wobei gleiche Bezugszeichen gleiche Merkmale bezeichnen. Wenn es die Übersichtlichkeit erfordert oder es anderweitig sinnvoll erscheint, wird auf bereits erwähnte Bezugszeichen in nachfolgenden Figuren verzichtet.

Es zeigen:

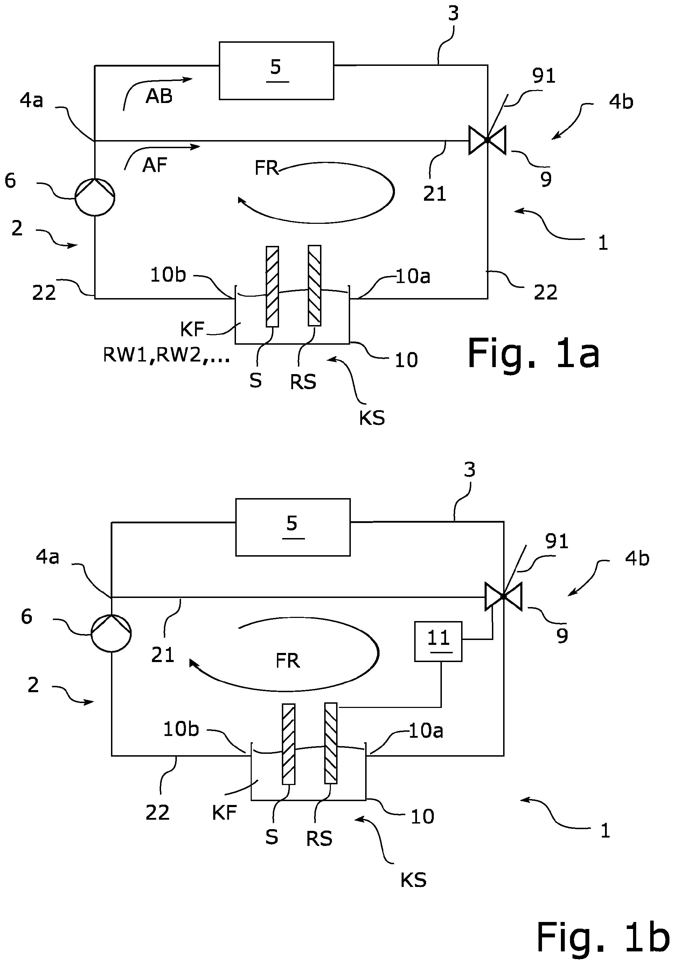

Fig. 1a: Eine erste Ausgestaltung der mobilen Anlage;
Fig. 1 b: Eine weitere Ausgestaltung der mobilen Anlage;
Fig. 2: Eine weitere Ausgestaltung der mobilen Anlage.

Fig. 1a zeigt eine erste Ausgestaltung der mobilen Anlage für das Kalibrieren, Verifizieren und/oder Justieren eines Sensors S. Die Anlage umfasst einen Förderkreislauf 1 mit einer Fluidleitung 2. Der Förderkreislauf 1 wird in dieser Ausgestaltung durch einen gemeinsam geführten zweiten Abschnitt 22, sowie einem ersten Abschnitt 21 der Fluidleitung 2 und einer dazu in einem Bypass geführten Bypass-Leitung 3 gebildet. An einer ersten Verbindungsstelle 4a verzweigt sich der zweite Abschnitt 22 in den ersten Abschnitt 21 und die Bypass-Leitung 3. Durch den Förderkreislauf 1 wird mittels einer Pumpe 6 in einer Förderrichtung FR (hier im Uhrzeigersinn) eine Kalibrierflüssigkeit KF gefördert, und zwar durch den ersten 21 und zweiten Abschnitt 22 und durch die Bypass-Leitung. Die Pumpe 6 ist dabei in dem zweiten Abschnitt 22 angeordnet. Sie ist zum Fördern einer Fördermenge von 100-1000 l/h ausgestaltet, bspw. etwa 300l/h wie etwa bei einer handelsüblichen Aquariumpumpe.

An einer zweiten Verbindungsstelle 4b münden der erste Abschnitt 21 und die Bypass-Leitung 3 wieder in den zweiten Abschnitt 22, so dass der Förderkreislauf 1 damit geschlossen ist. Es wird ein erster Anteil AB der Kalibrierflüssigkeit durch die Bypass-Leitung 3 und ein zweiter Anteil AF durch den ersten Abschnitt 21 gefördert. Nur der durch die Bypass-Leitung 3 geförderte Anteil AB wird mittels einer darin angeordneten Filtereinheit 5 fortlaufend entsalzt. Diese ist als ein Mischbettfilter ausgestaltet, welcher ein Kationen- und ein Anionenaustauschharz umfasst. Die Filtereinheit 5 ist sehr einfach auswechselbar, da es sich um eine zusammensteckbare Anlage handelt. Erfahrungswerte der Anmelderin zeigen, dass bei typischen Anwendungen die Filtereinheit 5 ca. bis zu 40 Mal für das Kalibrieren, Verifizieren und/oder Justieren eines Sensors S verwendet werden kann, bis ein Austausch erforderlich ist.

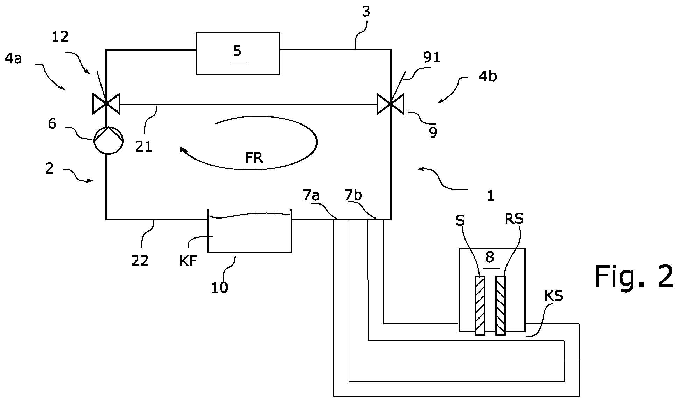

Der durch die Bypass-Leitung 3 geförderte und damit ständig entsalzte Anteil AB der Kalibrierflüssigkeit KF wird durch die Einstellung einer einstellbaren Ventileinheit 9 mit einem Regler 91 bestimmt. Der Regler 91 ist in der in Fig. 1a gezeigten Ausgestaltung manuell betätigbar bzw. einstellbar. Bevorzugt ist die Ventileinheit 9 nach der Erfindung an der zweiten Verbindungsstelle 4b d.h. an der stromabwärts-seitigen Verzweigung des Bypasses angeordnet, wodurch in der Bypass-Leitung 3 und dem ersten Abschnitt 21 derselbe Druck vorliegt. Alternativ ist es im Rahmen der Erfindung aber durchaus möglich, die Ventileinheit 9 an der ersten Verbindungsstelle 4a d.h. an der stromaufwärtsseitigen Verzweigung des Bypasses, anzuordnen. Ggf. ist es auch möglich, dass die mobile Anlage mehrere miteinander zusammenwirkende Ventileinheiten, d.h. die Ventileinheit 9 nach der Erfindung und eine weitere Ventileinheit 12 (in Fig. 2 gezeigt), aufweist.

Die mobile Anlage nach der Erfindung weist in dieser Ausgestaltung ferner eine Kalibrierstelle KS auf, an der der zu verifizierende und/oder zu justierende Sensor S sowie ein zur Messung der Prozessgröße dienender Referenzsensor RS in den Förderkreislauf 1 einbringbar ist.

Die Kalibrierstelle KS wird in dieser Ausgestaltung durch ein offenes Behältnis 10 gebildet, in das die Kalibrierflüssigkeit KF einfüllbar ist und in das beim Kalibrieren, Verifizieren und/oder Justieren des Sensors S der Sensor S sowie der Referenzsensor RS gemeinsam eintauchen und dadurch in den Förderkreislauf 1 eingebracht sind. Diese sind an eine (ggf. gemeinsame)Transmittereinheit (nicht dargestellt) angeschlossen, die der Verarbeitung und/oder Weiterleitung eines jeweils vom dem Sensor S sowie dem Referenzsensor RS erzeugten und von der Prozessgröße abhängigen Messsignals dient. Der vom Referenzsensor RS angezeigte Messwert wird als korrekt angenommen. Der Referenzsensor RS wird hierfür in der Praxis selbst in regelmäßigen Abständen, bspw. jährlich, kalibriert.

Das Behältnis 10 umfasst ein Volumen von ca. 1,5 Liter. Als Kalibierflüssigkeit KF wird als Ausgangsflüssigkeit bspw. voll- oder teilentsalztes Wasser verwendet. Das Behältnis 10 ist mittels einem in Bezug auf die Förderrichtung FR stromaufwärts angeordneten Einlass 10a und einem stromabwärts angeordneten Auslass 10b in den Förderkreislauf 1 integriert.

Bei einem offenen Behältnis 10 bildet ein Gas, das in der das offene Behältnis 10 umgebenden Umgebungsluft enthalten ist, mittels einer chemischen Reaktion zwischen dem Gas und der in der im offenen Behältnis 10 enthaltenen Kalibrierflüssigkeit KF Salze. Bspw. reagiert teil- oder vollentsalztes Wasser als Kalibrierflüssigkeit KF mit der Umgebungsluft, indem sich CO2 in der Kalibrierflüssigkeit KF anreichert und löst. Dadurch steigt der Salzgehalt und damit auch z.B. der Leitwert als Prozessgröße stetig an. Dadurch, dass gleichzeitig der die Bypass-Leitung 3 durchfließende Anteil AB der Kalibrierflüssigkeit KS im Förderkreislauf f1 fortlaufend entsalzt wird, kann dieser Effekt ausgeglichen werden. Dadurch kann der Salzgehalt an der Kalibrierstelle KS und damit die vom Salzgehalt abhängige Prozessgröße auf einen stabilen Referenzwert RW1;... eingestellt werden. Mit der mobilen Anlage ist als Referenzwert für die Prozessgröße bspw. ein sehr kleiner Leitwert von 0,5µS/cm (Mikro-Siemens pro cm) einstellbar.

Bei einer Förderung durch den Förderkreislauf 1 bestimmt die Einstellung der Ventileinheit 9 den Salzgehalt der Kalibrierflüssigkeit KF an der Kalibrierstelle KS. Daher ist mittels der Einstellung der Ventileinheit 9 die an der Kalibrierstelle KS vorliegende Prozessgröße auf den zumindest einen Referenzwert RW1;RW2,...einstellbar.

Bevorzugt wird im Falle einer Mehrpunkt-Kalibrierung die Ventileinheit 9 zunächst derart eingestellt (bspw. manuell) dass die Prozessgröße an der Kalibrierstelle KS einen ersten Referenzwert RW1 annimmt, wobei der Sensor S an diesem ersten Referenzwert RW1 kalibriert wird. Anschließend wird nur durch eine Umstellung der Ventileinheit 9 die Prozessgröße an der Kalibrierstelle KS auf einen zweiten Referenzwert RW2 gestellt und der Sensor S an diesem zweiten Referenzwert RW2 kalibriert (bzw. anschließend auf weitere Referenzwerte RW3,RW4,... etc.). Hierfür ist kein Austausch der Kalibrierflüssigkeit KF in der mobilen Anlage vonnöten, da im Wesentlichen nur die Ventileinheit 9 umgestellt werden. Der Sensor S und der Referenzsensor können dabei unverändert in der mobilen Anlage verbleiben.

In den in den Ausführungsbeispielen gezeigten Ausgestaltungen ist das Behältnis 10 als offen ausgestaltet. Selbstverständlich kann das Behältnis 10 nach der Erfindung alternativ aber auch als ein verschlossenes bzw. zumindest verschließbares Behältnis 10 ausgestaltet sein. In diesem Fall kann ausgehend von einem Ausgangs-Salzgehalt bzw. einer dazugehörigen Prozessgröße über eine Einstellung der Ventileinheit 9 zumindest der Salzgehalt an der Kalibrierstelle KS verringert werden bzw. die dazugehörigen Referenzwerte RW1,RW2, ... für die Prozessgröße eingestellt werden. Ggf. kann bei einem offenen oder verschließbaren Behältnis 10 auch einmalig Salz in das Behältnis 10 zugesetzt werden.

Fig. 1b zeigt die im Wesentlichen schon in Fig. 1a gezeigte mobile Anlage nach der Erfindung, wobei hier zusätzlich eine mit der Ventileinheit 9 verbundene Regel-/Auswerteeinheit 11, bspw. mit einem Mikroprozessor, vorgesehen ist. Die Regel-/Auswerteeinheit 11 ist mit dem Referenzsensor RS verbindbar. Mittels der Regel-/Auswerteeinheit 11 ist ein weitestgehend automatisiertes Kalibrieren, Verifizieren und/oder Justieren des Sensors S möglich.

In Fig. 2 ist eine weitere Ausgestaltung nach der Erfindung gezeigt. Auch diese umfasst das ggf. offene Behältnis 10 mit der Kalibrierflüssigkeit KF, welches hier aber nur als Reservoir für die Kalibrierflüssigkeit KF und nicht zum Einbringen des Sensors S und des Referenzsensors RS dient. In der in Fig. 2 gezeigten Ausgestaltung sind im zweiten Abschnitt 22 zwei Anschlüsse 7a,7b angeordnet. Über diese Anschlüsse 7a,7b ist eine als Kalibrierstelle KS dienende Wechselarmatur 8 in den Förderkreislauf integrierbar.

In den vorstehend genannten Industrien sind Wechselarmaturen weit verbreitet, mit denen Sensoren in ein Prozessmedium eingeführt und entnommen werden können, ohne dabei laufende Prozesse zu unterbrechen. Die Sensoren werden, manuell oder automatisch, axial zwischen einer Prozessstellung und einer Servicestellung der Wechselarmatur verfahren. Die in Fig. 2 gezeigte Wechselarmatur 8 ist dazu eingerichtet, zwei Sensoren, nämlich den zu kalibrierenden, zu verifizierenden und/oder zu justierenden Sensor S sowie den Referenzsensor RS aufzunehmen und in den Förderkreislauf 1 einzubringen. Alternativ kann die Kalibrierstelle KS auch zwei in einer Rohrleitung angeordnete Wechselarmaturen 8 aufweisen, die dazu ausgestaltet sind, jeweils den zu kalibrierenden, zu verifizierenden und/oder zu justierenden Sensor S und den Referenzsensor RS in den Förderkreislauf 1 einzubringen. Alternativ zu der in Fig. 2 gezeigten Ausgestaltung können die Anschlüsse 7a,7b zur Integration der Wechselarmatur 8 auch an anderen Stellen des zweiten Abschnitts 22, bspw. zwischen der Pumpe 6 und der ersten Verbindungsstelle 4a, angeordnet sein.

Selbstverständlich ist auch eine Kombination der in Fig. 2 und Fig. 1a,1b gezeigten Ausgestaltungen im Rahmen der Erfindung möglich. Beispielsweise kann einer der beiden Sensoren S, RS (d.h. der zu kalibrierende zu verifizierende und/oder zu justierende Sensor S und der Referenzsensor RS) in einem (offenen oder geschlossenen bzw. verschließbaren) Behältnis 10 angeordnet sein, und der andere der beiden Sensoren S,RS in der Wechselarmatur. In diesem Fall wird die Kalibriestelle KS sowohl von dem Behältnis 10, als auch von der Wechselarmatur 8 gebildet. Bspw. kann der zu kalibrierenden zu verifizierende und/oder zu justierende Sensor S mittels der Wechselarmatur 8, und der Referenzsensor mittels des Behältnis 10 in den Förderkreislauf 1 eingebracht sein. Letzterer Fall ist bspw. vorteilhaft, wenn der bestimmungsgemäße Einsatzort des Sensors S im Betrieb in der Wechselarmatur 8 ist. In diesem Fall kann der Sensor S zum Kalibrieren, Verifizieren und/oder Justieren an seinem bestimmungsgemäße Einsatzort verbleiben.

Die Fluidleitungen der zusammensteckbaren mobilen Anlage sind als PTFE-Kunststoffschläuche mit einem Durchmesser kleiner als 10 mm ausgestaltet Dadurch kann die gesamte mobile Anlage ein Gesamtgewicht unterhalb von 10 kg aufweisen, bspw. nur ca. 5kg.

Untersuchungen der Anmelderin zeigen, dass mit der mobilen Anlage eine Genauigkeit von +/- 2% beim Kalibrieren, Verifizieren und/oder Justieren des Sensors S erreicht wird. Eine derartig hohe Genauigkeit ist für die Leitfähigkeit als Prozessgröße mit den aus dem Stand der Technik bekannten Kalibrierlösungen nicht für alle in der Praxis verwendeten Messbereiche erfüllbar. Dies gilt besonders für kleinere Messbereiche mit einem maximalem Leitwert unterhalb von 50µS/cm, insbesondere unterhalb von 10 µS/cm.

### Bezugszeichen und Symbole

- 1: Förderkreislauf
- 2: Fluidleitung
- 21,22: erster, zweiter Abschnitt der Fluidleitung
- 3: Bypass-Fluidleitung
- 4a,4b: erste und zweite Verbindungsstelle
- 5: Filtereinheit
- 6: Pumpe
- 7a,7b: Anschlüsse
- 8: Wechselarmatur
- 9: Ventileinheit
- 91: Regler
- 10: Behältnis
- 10a,10b: Einlass, Auslass
- 11: Regel-/Auswerteeinheit
- 12: weitere Ventileinheit
- S: Sensor
- RS: Referenzsensor
- KF: Kalibrierflüssigkeit
- KS: Kalibrierstelle
- FR: Förderrichtung
- AB: durch Bypass-Fluidleitung geförderter Anteil
- AF: durch den ersten Abschnitt der Fluidleitung geförderter Anteil
- RW1,RW2,...: Referenzwerte

## Patentansprüche

1. Mobile Anlage für das Kalibrieren, Verifizieren und/oder Justieren eines Sensors (S) zur Bestimmung einer von einer Salzkonzentration abhängigen Prozessgröße eines Prozessmediums, umfassend einen Förderkreislauf (1), der zum Fördern einer darin eingebrachten Kalibrierflüssigkeit (KF) ausgestaltet ist, aufweisend:
- eine Fluidleitung (2);
- eine Bypass-Fluidleitung (3), die über eine erste Verbindungsstelle (4a) und eine zweite Verbindungsstelle (4b) in einem Bypass zu einem ersten Abschnitt (21) der Fluidleitung (2) mit der Fluidleitung (2) verbunden ist, mit einer Filtereinheit (5), die zum fortlaufenden Entsalzen eines durch die Bypass-Fluidleitung (3) geförderten Anteils der Kalibrierflüssigkeit (KF) ausgestaltet ist, und;
- eine in der Fluidleitung angeordnete Pumpe (6), die dazu ausgestaltet ist, die Kalibrierflüssigkeit (KF) in einer Förderrichtung (FR) durch den Förderkreislauf (1) zu fördern;
- einen Referenzsensor (RS), welcher zur Messung der Prozessgröße dient;
- eine Kalibrierstelle (KS), an welcher der Referenzsensor (RS) in den Förderkreislauf (1) eingebracht ist, und an welcher der zu kalibrierende, zu verifizierende und/oder zu justierende Sensor (S) einbringbar ist, wobei
- - die Kalibrierstelle (KS) in einem zweiten Abschnitt (22) der Fluidleitung (2) angeordnet ist, der vom ersten Abschnitt (21) der Fluidleitung verschieden ist,
oder
- - eine Wechselarmatur (8), welche als Kalibrierstelle (KS) dient, welche mittels in einem vom ersten Abschnitt (2) verschiedenen zweiten Abschnitt (22) der Fluidleitung (2) angeordneter Anschlüsse (7a,7b), in den Förderkreislauf (1) integriert ist;
und
- eine einstellbare Ventileinheit (9), mittels derer ein Verhältnis des durch die Bypass-Fluidleitung (3) geförderten Anteils (AB) der Kalibrierflüssigkeit (KF) zu einem durch den ersten Abschnitt (21) der Fluidleitung (2) geförderten Anteils (AF) der Kalibrierflüssigkeit einstellbar ist,
wobei mittels einer Einstellung, insb. Regelung, der Ventileinheit (9) die an der Kalibrierstelle (KS) vorliegende Prozessgröße auf zumindest einen vorgebbaren Referenzwert (RW1;RW2,...) für die Prozessgröße einstellbar, insb. regelbar, ist.

2. Mobile Anlage nach zumindest einem der vorherigen Ansprüche,
umfassend ein in dem zweiten Abschnitt (22) der Fluidleitung (2) angeordnetes Behältnis (10), das mit der Fluidleitung (2) verbunden ist, mittels eines Einlasses (10a) zum Einströmen der Kalibrierflüssigkeit (KF) aus dem zweiten Abschnitt (22) der Fluidleitung (2) in das Behältnis (10) und mittels eines Auslasses (10b) zum Ausströmen der Kalibrierflüssigkeit (KF) aus dem Behältnis (10) in den zweiten Abschnitt (22) der Fluidleitung (2).

3. Mobile Anlage nach Anspruch 2,
wobei das Behältnis (10) die Kalibrierstelle (KS) bildet, so dass beim Kalibrieren, Verifizieren und/oder Justieren des Sensors (S) der zu kalibrierende, zu verifizierende und/oder zu justierende Sensor (S) sowie der Referenzsensor (RS) in die in dem Behältnis (10) enthaltene Kalibrierflüssigkeit (KF) eintauchen.

4. Mobile Anlage nach Anspruch 2 oder 3,
wobei es sich um ein offenes Behältnis (10) handelt,
und wobei ein Gas, das in der das offene Behältnis (10) umgebenden Umgebungsluft enthalten ist, mittels einer chemischen Reaktion zwischen dem Gas und der in der im offenen Behältnis (10) enthaltenen Kalibrierflüssigkeit (KF) Salze in der Kalibrierflüssigkeit (KF) bildet.

5. Mobile Anlage nach zumindest einem der vorherigen Ansprüche,
wobei ein einstellbarer Regler (91) der Ventileinheit (9) die Ventileinheit dazu steuert, dass die an der Kalibrierstelle (KS) vorliegende Prozessgröße aufeinanderfolgend unterschiedliche vorgebbare Referenzwerte (RW1,RW1;..) für die Prozessgröße, insb. zumindest zwei unterschiedliche vorgebbare Referenzwerte (RW1,RW1;..) für die Prozessgröße, aufweist, während die Kalibrierflüssigkeit (KF), der zu kalibrierende, zu verifizierende und/oder zu justierende Sensor (S) und der als Referenz dienende Referenzsensor (RS) in dem Förderkreislauf (1) verbleiben.

6. Mobile Anlage nach zumindest einem der vorherigen Ansprüche,
wobei es sich bei der mobilen Anlage um eine mobile Anlage für das Kalibrieren, Verifizieren und/oder Justieren eines Leitfähigkeitssensors handelt, der insbesondere einen Messbereich aufweist, der durch einen maximalen Leitwert kleiner als 50 µS/cm, vorzugsweise kleiner als 10 µS/cm, begrenzt ist.

7. Mobile Anlage nach zumindest einem der vorherigen Ansprüche,
wobei die Pumpe (6) in dem zweiten Abschnitt (22) der Fluidleitung (2) angeordnet ist.

8. Mobile Anlage nach zumindest einem der vorherigen Ansprüche, wobei die Pumpe (6) angrenzend zu der ersten Verbindungstelle (4a) angeordnet ist,
in Bezug auf die Förderrichtung (FR) der Pumpe (6) im Wesentlichen stromaufwärts der ersten Verbindungsstelle (4a),
und wobei die Ventileinheit (9) an der zweiten Verbindungsstelle (4b) angeordnet ist.

9. Mobile Anlage nach zumindest einem der vorherigen Ansprüche, wobei als Kalibrierflüssigkeit (KF) zumindest teilentsalztes, insb. vollentsalztes Wasser, in den Förderkreislauf (1) eingefüllt ist.

10. Mobile Anlage nach zumindest einem der vorherigen Ansprüche, wobei der Regler (91) der Ventileinheit (9) und/oder die Ventileinheit (9) selbst manuell betätigbar ist.

11. Mobile Anlage nach zumindest einem der vorherigen Ansprüche,
umfassend eine Regel-/Auswerteeinheit (11), die den Regler (91) umfasst,
und wobei die Regel-/Auswerteeinheit (11) mit der Ventileinheit (91) verbunden und mit dem Referenzsensor (RS) verbindbar ist.

12. Mobile Anlage nach zumindest einem der vorherigen Ansprüche,
wobei die Komponenten der mobilen Anlage, insb. die Fluidleitung (2), die Bypass-Fluidleitung (3), die Filtereinheit (5), das Behältnis (10), die Pumpe (6) und die Ventileinheit (9), als ineinander zusammensteckbare Komponenten ausgestaltet sind.

13. Verfahren zum Kalibrieren, Verifizieren und/oder Justieren eines Sensors zur Bestimmung einer von einer Salzkonzentration abhängigen Prozessgröße eines Prozessmediums,
wobei eine mobile Anlage verwendet wird, umfassend einen Förderkreislauf (1), der zum Fördern einer darin eingebrachten Kalibrierflüssigkeit (KF) ausgestaltet ist, aufweisend:
- eine Fluidleitung (2);
- eine Bypass-Fluidleitung (3), die über eine erste Verbindungsstelle (4a) und eine zweite Verbindungsstelle (4b) in einem Bypass zu einem ersten Abschnitt (21) der Fluidleitung (2) mit der Fluidleitung (2) verbunden ist, mit einer Filtereinheit (5), die zum fortlaufenden Entsalzen eines durch die Bypass-Fluidleitung (3) geförderten Anteils der Kalibrierflüssigkeit (KF) ausgestaltet ist und;
- eine in der Fluidleitung (2) angeordnete Pumpe (6), die dazu ausgestaltet ist, die Kalibrierflüssigkeit (KF) in einer Förderrichtung (FR) durch den Förderkreislauf (1) zu fördern;
- eine einstellbare Ventileinheit (9), mittels derer ein Verhältnis des durch die Bypass-Fluidleitung (3) geförderten Anteils (AB) der Kalibrierflüssigkeit (KF) zu einem durch den ersten Abschnitt (21) der Fluidleitung (2) geförderten Anteil (AF) der Kalibrierflüssigkeit einstellbar ist,
wobei das Verfahren die Schritte umfasst:
- Einbringen des zu kalibrierenden, zu verifizierenden und/oder zu justierenden Sensors (S) sowie eines zur Messung der Prozessgröße dienenden Referenzsensors (RS) in den Förderkreislauf an einer Kalibrierstelle (KS);
- Einbringen des zu kalibrierenden, zu verifizierenden und/oder zu justierenden Sensors (S) sowie eines zur Messung der Prozessgröße dienenden Referenzsensors (RS) in den Förderkreislauf (1) an einer im zweiten Abschnitt (22) der Fluidleitung (2) angeordneten Kalibrierstelle (KS) und/oder in zumindest eine, als Kalibrierstelle (KS) dienende Wechselarmatur (8), die mittels im zweiten Abschnitt (22) der Fluidleitung (2) angeordneter Anschlüsse (7a,7b) in den Förderkreislauf (1) der mobilen Anlage integriert ist;
- Einstellen, insb. Regeln, der Ventileinheit (9) derart, dass die an der Kalibrierstelle (KS) vorliegende Prozessgröße einen vorgebbaren Referenzwert (RW1;RW2...) für die Prozessgröße aufweist.

14. Verfahren nach Anspruch 13,
wobei das Einstellen, insb. Regeln, der Ventileinheit (9) dadurch erfolgt, dass ein mit dem Referenzsensor (RS) ermittelter Messwert der Prozessgröße mit dem vorgebbaren Referenzwert (RW1;RW2,..) übereinstimmt.

15. Verfahren nach zumindest einem der vorherigen Ansprüche 13 bis 14,
umfassend:
Wiederkehrendes Einstellen, insb. Regeln, der Ventileinheit (9) derart, dass die an der Kalibrierstelle (KS) vorliegenden Prozessgröße zu aufeinanderfolgenden Zeitpunkten unterschiedliche vorgebbare Referenzwerte (RW1,RW2,...) für die Prozessgröße, insb. zumindest zwei unterschiedliche vorgebbare Referenzwerte (RW1,RW2,...) für die Prozessgröße, aufweist,
wobei die Kalibrierflüssigkeit (KF), der zu kalibrierende, zu verifizierende und/oder zu justierende Sensor (S) und der als Referenz dienende Referenzsensor (RS) zu den aufeinanderfolgenden Zeitpunkten während dem wiederkehrenden Einstellen, insb. Regeln, der Ventileinheit (9) in dem Förderkreislauf (1) verbleiben.

## Claims

1. A mobile system for calibrating, verifying and/or adjusting a sensor (S) for determining a process variable of a process medium that is dependent on a salt concentration, comprising a conveying circuit (1) configured to convey a calibration liquid (KF) introduced therein, having:
- a fluid line (2);
- a bypass fluid line (3), which is connected to the fluid line (2) via a first connection point (4a) and a second connection point (4b), in a bypass to a first section (21) of the fluid line (2), with a filter unit (5) configured for the continuous desalination of a portion of the calibration liquid (KF) conveyed through the bypass fluid line (3), and;
- a pump (6) disposed in the fluid line and configured to convey the calibration liquid (KF) in a conveying direction (FR) through the conveying circuit (1);
- a reference sensor (RS) which serves to measure the process variable;
- a calibration site (KS) at which the reference sensor (RS) is included into the conveying circuit (1) and at which the sensor (S) to be calibrated, verified and/or adjusted can be included, wherein
-- the calibration site (KS) is disposed in a second section (22) of the fluid line (2), which differs from the first section (21) of the fluid line, or
-- an interchangeable fitting (8) which serves as the calibration site (KS) and which is integrated into the conveying circuit (1) by means of connections (7a, 7b) disposed in a second section (22) of the fluid line (2), which differs from the first section (2),
and
- an adjustable valve unit (9) by means of which a ratio of the portion (AB) of the calibration liquid (KF) conveyed through the bypass fluid line (3) to a portion (AF) of the calibration liquid conveyed through the first section (21) of the fluid line (2) can be set;
wherein, by setting, in particular controlling, the valve unit (9), the process variable present at the calibration site (KS) can be set, in particular controlled, to at least one specifiable reference value (RW1;RW2,...) for the process variable.

2. The mobile system as claimed in at least one of the preceding claims,
comprising a container (10) disposed in the second section (22) of the fluid line (2) and connected to the fluid line (2) by means of an inlet (10a) for the inflow of the calibration liquid (KF) from the second section (22) of the fluid line (2) into the container (10) and by means of an outlet (10b) for the outflow of the calibration liquid (KF) from the container (10) into the second section (22) of the fluid line (2).

3. The mobile system as claimed in claim 2,
wherein the container (10) forms the calibration site (KS) such that, during the calibration, verification and/or adjustment of the sensor (S), the sensor (S) and the reference sensor (RS) to be calibrated, verified and/or adjusted are in dipped into the calibration liquid (KF) contained in the container (10).

4. The mobile system as claimed in claim 2 or 3,
wherein it is an open container (10),
and wherein a gas contained in the ambient air surrounding the open container (10) forms salts in the calibration liquid (KF) by means of a chemical reaction between the gas and the calibration liquid (KF) contained in the open container (10).

5. The mobile system as claimed in at least one of the preceding claims,
wherein an adjustable controller (91) of the valve unit (9) controls the valve unit such that the process variable present at the calibration site (KS) has successively different specifiable reference values (RW1;RW2,...) for the process variable, in particular at least two different specifiable reference values (RW1;RW2,...) for the process variable, while the calibration liquid (KF), the sensor (S) to be calibrated, verified and/or adjusted and the reference sensor (RS) serving as a reference remain in the conveying circuit (1).

6. The mobile system as claimed in at least one of the preceding claims,
wherein the mobile system is a mobile system for calibrating, verifying and/or adjusting a conductivity sensor having, in particular, a measuring range that is delimited by a maximum conductance of less than 50 µS/cm, preferably less than 10 µS/cm.

7. The mobile system as claimed in at least one of the preceding claims,
wherein the pump (6) is disposed in the second section (22) the fluid line (2).

8. The mobile system as claimed in at least one of the preceding claims, wherein the pump (6) is disposed adjacent to the first connection point (4a)
substantially upstream of the first connection point (4a) with respect to the conveying direction (FR) of the pump (6),
and wherein the valve unit (9) is disposed at the second connection point (4b).

9. The mobile system as claimed in at least one of the preceding claims, wherein at least partially desalinated water, in particular completely desalinated water, is added into the conveying circuit (1) as calibration liquid (KF).

10. The mobile system as claimed in at least one of the preceding claims, wherein the controller (91) of the valve unit (9) and/or the valve unit (9) itself can be manually actuated.

11. The mobile system as claimed in at least one of the preceding claims,
comprising a control/evaluation unit (11) which comprises the controller (91),
and wherein the control/evaluation unit (11) is connected to the valve unit (91) and can be connected to the reference sensor (RS).

12. The mobile system as claimed in at least one of the preceding claims,
wherein the components of the mobile system, in particular the fluid line (2), the bypass fluid line (3), the filter unit (5), the container (10), the pump (6) and the valve unit (9), are configured to be components than can be snap-fitted inside each other.

13. A method for calibrating, verifying and/or adjusting a sensor for determining a process variable of a process medium depending on a salt concentration,
wherein use is made of a mobile system comprising a conveying circuit (1) configured to convey a calibration liquid (KF) introduced therein, having:
- a fluid line (2);
- a bypass fluid line (3), which is connected to the fluid line (2) via a first connection point (4a) and a second connection point (4b), in a bypass to a first section (21) of the fluid line (2), with a filter unit (5) configured for the continuous desalination of a portion of the calibration liquid (KF) conveyed through the bypass fluid line (3), and;
- a pump (6) disposed in the fluid line and configured to convey the calibration liquid (KF) in a conveying direction (FR) through the conveying circuit (1);
- an adjustable valve unit (9) by means of which a ratio of the portion (AB) of the calibration liquid (KF) conveyed through the bypass fluid line (3) to a portion (AF) of the calibration liquid conveyed through the first section (21) of the fluid line (2) can be set,
wherein the method comprises the following steps:
- introducing the sensor (S) to be calibrated, verified and/or adjusted and a reference sensor (RS) serving to measure the process variable into the conveying circuit at a calibration site (KS);
- introducing the sensor (S) to be calibrated, verified and/or adjusted and a reference sensor (RS) serving to measure the process variable into the conveying circuit (1) at a calibration site (KS) disposed in the second section (22) of the fluid line (2) and/or into at least one interchangeable fitting (8) serving as a calibration site (KS), which is integrated into the conveying circuit (1) of the mobile system by means of connections (7a, 7b) disposed in the second section (22) of the fluid line (2);
- setting, in particular controlling, the valve unit (9) such that the process variable present at the calibration site (KS) has a specifiable reference value (RW1;RW2,...) for the process variable.

14. The method as claimed in claim 13,
wherein the setting, in particular controlling, of the valve unit (9) is carried out by a measured value of the process variable determined with the reference sensor (RS) matching the specifiable reference value (RW1;RW2,...).

15. The method as claimed in at least one of the preceding claims 13 to 14,
comprising:
recurrently setting, in particular controlling, the valve unit (9) such that the process variable present at the calibration site (KS) at successive instants has different specifiable reference values (RW1;RW2,...) for the process variable, in particular at least two different specifiable reference values (RW1;RW2,...) for the process variable,
wherein the calibration liquid (KF), the sensor (S) to be calibrated, verified and/or adjusted and the reference sensor (RS) serving as a reference remain in the conveying circuit (1) at the successive instants during the recurrent setting, in particular controlling, of the valve unit (9).

## Revendications

1. Installation mobile pour l'étalonnage, la vérification et l'ajustage d'un capteur (S) destiné à la détermination d'une grandeur de process dépendant d'une concentration en sel d'un produit de process, laquelle installation comprend un circuit de transport (1), lequel est conçu pour transporter un liquide d'étalonnage (KF) qui y est introduit, laquelle installation comprend :
- une conduite de fluide (2) ;
- une conduite de fluide de dérivation (3) reliée à la conduite de fluide (2) par l'intermédiaire d'un premier point de liaison (4a) et d'un deuxième point de liaison (4b) en dérivation par rapport à une première section (21) de la conduite de fluide (2), avec une unité de filtrage (5) conçue pour dessaler en continu une partie du liquide d'étalonnage (KF) transporté par la conduite de fluide de dérivation (3), et ;
- une pompe (6) disposée dans la conduite de fluide, laquelle est conçue pour transporter le liquide d'étalonnage (KF) dans une direction de transport (FR) à travers le circuit de transport (1) ;
- un capteur de référence (RS), lequel sert à mesurer la grandeur de process ;
- un point d'étalonnage (KS), au niveau duquel le capteur de référence (RS) est inséré dans le circuit de transport (1), et au niveau duquel le capteur (S) à étalonner, à vérifier et/ou à ajuster peut être inséré,
- - le point d'étalonnage (KS) étant disposé dans une deuxième section (22) de la conduite de fluide (2), laquelle est différente de la première section (21) de la conduite de fluide, ou
- - un support rétractable (8) servant de point d'étalonnage (KS), lequel support est intégré dans le circuit de transport (1) au moyen de raccords (7a, 7b) disposés dans une deuxième section (22) de la conduite de fluide (2), différente de la première section (2) ; et
- une unité de vanne réglable (9), au moyen de laquelle un rapport entre la part (AB) du liquide d'étalonnage (KF) transportée par la conduite de fluide de dérivation (3) et une part (AF) du liquide d'étalonnage transportée par la première section (21) de la conduite de fluide (2) peut être réglé,
un réglage, notamment une régulation, de l'unité de vanne (9) permettant de régler, notamment de réguler, la grandeur de process disponible au point d'étalonnage (KS) à au moins une valeur de référence (RW1, RW2, ...) pouvant être définie pour la grandeur de process.

2. Installation mobile selon au moins l'une des revendications précédentes,
laquelle installation comprend un récipient (10) disposé dans la deuxième section (22) de la conduite de fluide (2) et relié à la conduite de fluide (2) au moyen d'une entrée (10a) pour l'écoulement du liquide d'étalonnage (KF) à partir de la deuxième section (22) de la conduite de fluide dans le récipient (10) et au moyen d'une sortie (10b) pour l'écoulement du liquide d'étalonnage (KF) à partir du récipient (10) dans la deuxième section (22) de la conduite de fluide (2).

3. Installation mobile selon la revendication 2,
pour laquelle le récipient (10) forme le point d'étalonnage (KS), de sorte que lors de l'étalonnage, de la vérification et/ou de l'ajustement du capteur (S), le capteur (S) à étalonner, à vérifier et/ou à ajuster ainsi que le capteur de référence (RS) sont immergés dans le liquide d'étalonnage (KF) contenu dans le récipient (10).

4. Installation mobile selon la revendication 2 ou 3,
pour laquelle il s'agit d'un récipient ouvert (10),
et pour laquelle un gaz contenu dans l'air ambiant entourant le récipient ouvert (10) forme des sels dans le liquide d'étalonnage (KF) au moyen d'une réaction chimique entre le gaz et le liquide d'étalonnage (KF) contenu dans le récipient ouvert (10).

5. Installation mobile selon au moins l'une des revendications précédentes, pour laquelle un régulateur réglable (91) de l'unité de vanne (9) commande l'unité de vanne de telle sorte que la grandeur de process disponible au point d'étalonnage (KS) présente successivement différentes valeurs de référence (RW1, RW2, ...) prédéfinissables pour la grandeur de process, notamment au moins deux valeurs de référence (RW1, RW2, ...) prédéfinissables différentes pour la grandeur de process, tandis que le liquide d'étalonnage (KF), le capteur (S) à étalonner, à vérifier et/ou à ajuster et le capteur de référence (RS), lequel sert de référence, restent dans le circuit de transport (1).

6. Installation mobile selon au moins l'une des revendications précédentes,
laquelle installation mobile est une installation mobile destinée à l'étalonnage, la vérification et/ou l'ajustement d'un capteur de conductivité, lequel capteur présente notamment une gamme de mesure limitée par une conductance maximale inférieure à 50 µS/cm, de préférence inférieure à 10 µS/cm.

7. Installation mobile selon au moins l'une des revendications précédentes, pour laquelle la pompe (6) est disposée dans la deuxième section (22) de la conduite de fluide (2).

8. Installation mobile selon au moins l'une des revendications précédentes, pour laquelle la pompe (6) est disposée de manière adjacente au premier point de liaison (4a), pour l'essentiel en amont du premier point de liaison (4a) par rapport à la direction de transport (FR) de la pompe (6),
et pour laquelle l'unité de vanne (9) est disposée au niveau du deuxième point de liaison (4b).

9. Installation mobile selon au moins l'une des revendications précédentes,
pour laquelle de l'eau au moins partiellement déminéralisée, notamment de l'eau entièrement déminéralisée, est introduite dans le circuit de transport (1) en tant que liquide d'étalonnage (KF).

10. Installation mobile selon au moins l'une des revendications précédentes,
pour laquelle le régulateur (91) de l'unité de vanne (9) et/ou l'unité de vanne (9) elle-même peut être actionné(e) manuellement.

11. Installation mobile selon au moins l'une des revendications précédentes,
laquelle installation comprend une unité de régulation/d'exploitation (11) comprenant le régulateur (91), et l'unité de régulation/d'exploitation (11) étant reliée à l'unité de vanne (91) et pouvant être reliée au capteur de référence (RS).

12. Installation mobile selon au moins l'une des revendications précédentes,
pour laquelle les composants de l'installation mobile, notamment la conduite de fluide (2), la conduite de fluide de dérivation (3), l'unité de filtrage (5), le récipient (10), la pompe (6) et l'unité de vanne (9), sont conçus sous la forme de composants pouvant être emboîtés les uns dans les autres.

13. Procédé pour l'étalonnage, la vérification et l'ajustage d'un capteur destiné à la détermination d'une grandeur de process dépendant d'une concentration en sel d'un produit de process,
procédé dans lequel on utilise une installation mobile comprenant un circuit de transport (1), lequel est conçu pour transporter un liquide d'étalonnage (KF) qui y est introduit, lequel circuit comprend :
- une conduite de fluide (2) ;
- une conduite de fluide de dérivation (3), laquelle est reliée à la conduite de fluide (2) par l'intermédiaire d'un premier point de liaison (4a) et d'un deuxième point de liaison (4b) en dérivation par rapport à une première section (21) de la conduite de fluide (2), avec une unité de filtrage (5), laquelle est conçue pour dessaler en continu une partie du liquide d'étalonnage (KF) transporté à travers la conduite de fluide de dérivation (3) et ;
- une pompe (6) disposée dans la conduite de fluide (2), laquelle est conçue pour transporter le liquide d'étalonnage (KF) dans une direction de transport (FR) à travers le circuit de transport (1) ;
- une unité de vanne réglable (9) au moyen de laquelle un rapport entre la partie (AB) du liquide d'étalonnage (KF) transportée par la conduite de fluide de dérivation (3) et une partie (AF) du liquide d'étalonnage transportée par la première section (21) de la conduite de fluide (2) peut être réglé,
le procédé comprenant les étapes consistant à :
- insérer le capteur (S) à étalonner, à vérifier et/ou à ajuster, ainsi qu'un capteur de référence (RS) servant à mesurer la grandeur de process, dans le circuit de transport en un point d'étalonnage (KS) ;
- insérer le capteur (S) à étalonner, à vérifier et/ou à ajuster, ainsi qu'un capteur de référence (RS) servant à mesurer la grandeur de process, dans le circuit de transport (1) en un point d'étalonnage (KS) disposé dans la deuxième section (22) de la conduite de fluide (2) et/ou dans au moins un support rétractable (8) servant de point d'étalonnage (KS), lequel support est intégré dans le circuit de transport (1) de l'installation mobile au moyen de raccords (7a, 7b) disposés dans la deuxième section (22) de la conduite de fluide (2) ;
- régler, notamment réguler, l'unité de vanne (9) de telle sorte que la grandeur de process disponible au point d'étalonnage (KS) présente une valeur de référence (RW1, RW2, ...) prédéfinissable pour la grandeur de process.

14. Procédé selon la revendication 13,
pour lequel le réglage, notamment la régulation, de l'unité de vanne (9) s'effectue par le fait qu'une valeur mesurée de la grandeur de process déterminée par le capteur de référence (RS) coïncide avec la valeur de référence (RW1, RW2, ...) prédéfinissable.

15. Procédé selon au moins l'une des revendications précédentes 13 à 14,
lequel procédé comprend :
Le réglage, notamment la régulation, récurrent de l'unité de vanne (9) de telle sorte que la grandeur de process disponible au point d'étalonnage (KS) présente à des moments successifs des valeurs de référence prédéfinissables différentes (RW1, RW2, ...) pour la grandeur de process, notamment au moins deux valeurs de référence (RW1, RW2, ...) prédéfinissables différentes pour la grandeur de process,
le liquide d'étalonnage (KF), le capteur (S) à étalonner, à vérifier et/ou à ajuster et le capteur de référence (RS), lequel sert de référence, restant dans le circuit de transport (1) aux moments successifs pendant le réglage, notamment la régulation, récurrent de l'unité de vanne (9).
